# EUROPEAN PATENT APPLICATION

(11) **EP 4 772 113 A2**
(43) Date of publication of application: **08.07.2026**
(21) Application number: 25227461.8
(22) Date of filing: 29.12.2025
(51) Int. Cl.: A61B 6/10

(54) **OBSTACLE PROCESSING METHOD AND APPARATUS FOR MEDICAL IMAGING SYSTEM, ELECTRONIC DEVICE, STORAGE MEDIUM, AND PROGRAM PRODUCT**

(30) Priority: 02.01.2025 CN 202510006193
(71) Applicant: GE Precision Healthcare LLC, Waukesha, WI 53188 (US)
(72) Inventor: WANG, Yan, Waukesha, 53188 (US); ZHU, Chao, Waukesha, 53188 (US); WANG, Chunzhu, Waukesha, 53188 (US); HUANG, Enquan, Waukesha, 53188 (US)
(74) Representative: Kilburn & Strode LLP

(57) **Abstract**

The present disclosure relates to an obstacle processing method and apparatus for a medical imaging system, an electronic device, a storage medium, and a program product. The method includes: collecting, by means of a visible light camera, a camera image corresponding to a target workspace of a target subsystem of a medical imaging system, wherein the target workspace is a target movement path or an imaging beam range; detecting an obstacle in the target workspace based on the camera image to obtain an obstacle detection result of the target workspace; and performing obstacle processing on the target workspace in response to the obstacle detection result indicating that an obstacle is detected.

## Description

### TECHNICAL FIELD

The present disclosure relates to the field of computer technology, and in particular, to an obstacle processing method for a medical imaging system, an obstacle processing apparatus for a medical imaging system, an electronic device, a computer-readable storage medium, and a computer program product.

### BACKGROUND

During the positioning process of a medical imaging system, unintended collisions with obstacles or patients frequently occur due to the automatic movement of subsystems. For example, in an X-ray imaging system, while an X-ray emission tube is tracking movement of a chest stand, the X-ray emission tube may collide with a tabletop or a patient. While the chest stand is tracking the X-ray emission tube in a reverse direction, a housing of the chest stand may collide with a patient. Such collisions may not only cause equipment damage, but also may cause harm to the patient, affecting the smooth progress of the medical procedure.

In addition, when the medical imaging system collects a medical image, if an obstacle is present in the imaging beam range, it may cause image artifacts. For example, when an X-ray imaging system is performing exposure for image capture, if a foreign object is present within the X-ray fan beam range, it may cause image artifacts. This not only reduces imaging quality and affects diagnostic accuracy, but may also result in the need for repeated exposures, thereby increasing the risk of radiation exposure for patients and medical personnel.

### SUMMARY OF THE INVENTION

The present disclosure provides a technical solution for obstacle processing for a medical imaging system.

According to an aspect of the present disclosure, provided is an obstacle processing method for a medical imaging system, comprising:
collecting, by means of a visible light camera, a camera image corresponding to a target workspace of a target subsystem of a medical imaging system, wherein the target workspace is a target movement path or an imaging beam range;
detecting an obstacle in the target workspace based on the camera image to obtain an obstacle detection result of the target workspace; and
performing obstacle processing on the target workspace in response to the obstacle detection result indicating that an obstacle is detected.

In a possible implementation, the collection, by means of a visible light camera, of a camera image corresponding to a target workspace of a target subsystem of a medical imaging system comprises:
collecting, by means of the visible light camera, a panoramic camera image corresponding to the medical imaging system, wherein the panoramic camera image covers the target workspace of the target subsystem of the medical imaging system.

In a possible implementation, the camera image comprises:
RGB color information and depth information;
   or,
RGB color information.

In a possible implementation, the method further comprises:
in response to the presence of an occluding object in the camera image, controlling the visible light camera to move, and re-collecting the camera image by means of the visible light camera.

In a possible implementation, the detection of an obstacle in the target workspace based on the camera image to obtain an obstacle detection result of the target workspace comprises:
inputting the camera image into a pre-trained obstacle detection model, and detecting, by means of the obstacle detection model, the obstacle in the target workspace; and
determining the obstacle detection result of the target workspace based on a detection result of the obstacle detection model.

In a possible implementation, the determination of the obstacle detection result of the target workspace based on a detection result of the obstacle detection model comprises:
recognizing, by means of a pre-trained position recognition model, a position of an object of a preset type in the target workspace to obtain a position recognition result of the object of the preset type; and
determining the obstacle detection result of the target workspace based on the detection result of the obstacle detection model and the position recognition result of the object of the preset type.

In a possible implementation, the position recognition model recognition comprises a tabletop position recognition model and/or a personnel position recognition model, and the preset type comprises tabletop and/or personnel.

In a possible implementation, the performing obstacle processing on the target workspace comprises:
displaying a bounding box surrounding the obstacle in a video stream of the visible light camera according to coordinates of the obstacle.

In a possible implementation, the performing obstacle processing on the target workspace comprises:
outputting obstacle warning information.

In a possible implementation, in a case where the target workspace is the target movement path, the performing obstacle processing on the target workspace comprises:
controlling the target subsystem to pause movement in response to a distance between the target subsystem and the obstacle being less than or equal to a preset distance, and controlling the target subsystem to continue moving along the target movement path in response to the obstacle being removed;
   or,
generating an alternative movement path for the target movement path, and controlling the target subsystem to move along the alternative movement path, wherein no obstacle exists on the alternative movement path;
   or,
controlling the obstacle to move so as to avoid the target movement path in response to the obstacle being a movable subsystem of the medical imaging system and the obstacle being in a movement-allowable state.

In a possible implementation, in a case where the target workspace is the imaging beam range, the performing obstacle processing on the target workspace comprises:
controlling the medical imaging system to pause medical image collection; and
in response to the obstacle being removed, allowing control of the medical imaging system to continue the medical image collection.

In a possible implementation, the medical imaging system is an X-ray imaging system; and
the target subsystem comprises at least one of: an X-ray emission subsystem, an X-ray detection subsystem, and a patient support subsystem.

In a possible implementation, the obstacle comprises at least one of the following types: other subsystems of the medical imaging system, a patient, a technician or medical personnel, and other objects in a space where the medical imaging system is located.

According to one aspect of the present disclosure, provided is an obstacle processing apparatus for a medical imaging system, comprising:
a collection module, configured to collect, by means of a visible light camera, a camera image corresponding to a target workspace of a target subsystem of a medical imaging system, wherein the target workspace is a target movement path or an imaging beam range;
a detection module, configured to detect an obstacle in the target workspace based on the camera image to obtain an obstacle detection result of the target workspace; and
a processing module, configured to perform obstacle processing on the target workspace in response to the obstacle detection result indicating that an obstacle is detected.

In a possible implementation, the collection module is configured to:
collecting, by means of the visible light camera, a panoramic camera image corresponding to the medical imaging system, wherein the panoramic camera image covers the target workspace of the target subsystem of the medical imaging system.

In a possible implementation, the camera image comprises:
RGB color information and depth information;
   or,
RGB color information.

In a possible implementation, the apparatus further includes:
a moving module, configured to, in response to the presence of an occluding object in the camera image, control the visible light camera to move, and re-collect the camera image by means of the visible light camera.

In a possible implementation, the detection module is configured to:
inputting the camera image into a pre-trained obstacle detection model, and detecting, by means of the obstacle detection model, the obstacle in the target workspace; and
determine the obstacle detection result of the target workspace based on a detection result of the obstacle detection model.

In a possible implementation, the detection module is configured to:
recognizing, by means of a pre-trained position recognition model, a position of an object of a preset type in the target workspace to obtain a position recognition result of the object of the preset type; and
determine the obstacle detection result of the target workspace based on the detection result of the obstacle detection model and the position recognition result of the object of the preset type.

In a possible implementation, the position recognition model recognition comprises a tabletop position recognition model and/or a personnel position recognition model, and the preset type comprises tabletop and/or personnel.

In a possible implementation, the processing module is configured to:
display a bounding box surrounding the obstacle in a video stream of the visible light camera according to coordinates of the obstacle.

In a possible implementation, the processing module is configured to:
output obstacle warning information.

In a possible implementation, in a case where the target workspace is the target movement path, the processing module is configured to:
control the target subsystem to pause movement in response to a distance between the target subsystem and the obstacle being less than or equal to a preset distance, and control the target subsystem to continue moving along the target movement path in response to the obstacle being removed;
   or,
generate an alternative movement path for the target movement path, and control the target subsystem to move along the alternative movement path, wherein no obstacle exists on the alternative movement path;
   or,
control the obstacle to move so as to avoid the target movement path in response to the obstacle being a movable subsystem of the medical imaging system and the obstacle being in a movement-allowable state.

In a possible implementation, in a case where the target workspace is the imaging beam range, the processing module is configured to:
control the medical imaging system to pause medical image collection; and
in response to the obstacle being removed, allow control of the medical imaging system to continue the medical image collection.

In a possible implementation, the medical imaging system is an X-ray imaging system; and
the target subsystem comprises at least one of: an X-ray emission subsystem, an X-ray detection subsystem, and a patient support subsystem.

In a possible implementation, the obstacle comprises at least one of the following types: other subsystems of the medical imaging system, a patient, a technician or medical personnel, and other objects in a space where the medical imaging system is located.

According to an aspect of the present disclosure, provided is an electronic device, comprising: one or more processors; and a memory for storing executable instructions, wherein the one or more processors are configured to invoke the executable instructions stored by the memory to perform the above method.

According to an aspect of the present disclosure, provided is a computer-readable storage medium, having computer program instructions stored thereon, wherein the computer program instructions, when executed by a processor, implement the above method.

According to an aspect of the present disclosure, provided is a computer program product, comprising computer-readable code or a non-volatile computer-readable storage medium bearing computer-readable code, wherein when the computer-readable code is run in an electronic device, a processor in the electronic device performs the above method.

In the embodiments of the present disclosure, a camera image corresponding to a target workspace of a target subsystem of a medical imaging system is collected by means of a visible light camera, where the target workspace is a target movement path or an imaging beam range; an obstacle in the target workspace is detected based on the camera image to obtain an obstacle detection result of the target workspace; and obstacle processing is performed on the target workspace in response to the obstacle detection result indicating that an obstacle is detected. Thereby, obstacle detection and obstacle processing are performed on the movement path or the imaging beam range of the subsystem of the medical imaging system based on the camera image collected by the visible light camera, which can reduce the use of sensors for obstacle detection, and lower the cost of obstacle processing for the medical imaging system. By detecting obstacles on the moving path of the subsystem of the medical imaging system and performing collision avoidance processing, the probability that the subsystem of the medical imaging system collides with a patient or other obstacles can be reduced, thereby reducing damage to the medical imaging system due to collisions and lowering the probability of injury to patients and other personnel by the medical imaging system. By detecting obstacles within the imaging beam range and performing obstacle processing, artifacts in medical images can be reduced, the imaging quality of medical images can be improved, the accuracy of diagnosis based on medical images can be improved, and repeated exposure can be reduced, thereby lowering radiation exposure risks for patients and medical personnel.

It should be understood that the foregoing general description and the following detailed description are merely exemplary and explanatory and do not limit the present disclosure.

Other features and aspects of the present disclosure will become apparent from the following detailed description of exemplary embodiments provided with reference to the accompanying drawings.

### BRIEF DESCRIPTION OF THE DRAWINGS

The accompanying drawings here are incorporated into the specification, constitute a part of the specification, illustrate embodiments compliant with the present disclosure, and are used together with the specification to describe the technical solutions of the present disclosure.
FIG. 1 shows a flowchart of an obstacle processing method for a medical imaging system provided in an embodiment of the present disclosure.
FIG. 2 shows a schematic view of an X-ray imaging system.
FIG. 3 shows another schematic view of the X-ray imaging system.
FIG. 4 shows a schematic view of a patient support subsystem.
FIG. 5 shows a block diagram of an obstacle processing apparatus for a medical imaging system provided in an embodiment of the present disclosure.
FIG. 6 shows a block diagram of an electronic device 1900 provided in an embodiment of the present disclosure.

### DETAILED DESCRIPTION

Various exemplary embodiments, features, and aspects of the present disclosure will be described in detail below with reference to the accompanying drawings. The same reference numerals in the drawings represent elements having the same or similar functions. While various aspects of the embodiments are illustrated in the accompanying drawing, the accompanying drawings are not necessarily drawn to scale unless specifically stated.

The word "exemplary" dedicated herein means "used as an example or an embodiment, or is illustrative". Any embodiment illustrated herein as "exemplary" is not necessarily interpreted as being superior to or better than other embodiments.

The term "and/or" herein is merely to describe the associations of associated objects, indicating that there may be three kinds of relationships. For example, A and/or B may indicate three situations, i.e., A exists alone, A and B exist simultaneously, or B exists alone. In addition, the term "at least one" herein indicates any one of a plurality or any combination of at least two of a plurality. For example, at least one of A, B, and C may indicate any one or more elements selected from the group consisting of A, B, and C.

In addition, in order to better illustrate the present disclosure, numerous specific details are given in the detailed description below. It should be understood by those skilled in the art that the present disclosure can be implemented without some of the specific details. In some examples, the methods, means, elements, and circuits well known to those skilled in the art are not described in detail in order to highlight the main idea of the present disclosure.

The embodiments of the present disclosure provide an obstacle processing method for a medical imaging system, in which a camera image corresponding to a target workspace of a target subsystem of a medical imaging system is collected by means of a visible light camera, where the target workspace is a target movement path or an imaging beam range; an obstacle in the target workspace is detected based on the camera image to obtain an obstacle detection result of the target workspace; and obstacle processing is performed on the target workspace in response to the obstacle detection result indicating that an obstacle is detected. Thereby, obstacle detection and obstacle processing are performed on the movement path or the imaging beam range of the subsystem of the medical imaging system based on the camera image collected by the visible light camera, which can reduce the use of sensors for obstacle detection, and lower the cost of obstacle processing for the medical imaging system. By detecting obstacles on the moving path of the subsystem of the medical imaging system and performing collision avoidance processing, the probability that the subsystem of the medical imaging system collides with a patient or other obstacles can be reduced, thereby reducing damage to the medical imaging system due to collisions and lowering the probability of injury to patients and other personnel by the medical imaging system. By detecting obstacles within the imaging beam range and performing obstacle processing, artifacts in medical images can be reduced, the imaging quality of medical images can be improved, the accuracy of diagnosis based on medical images can be improved, and repeated exposure can be reduced, thereby lowering radiation exposure risks for patients and medical personnel.

An obstacle processing method for a medical imaging system provided in an embodiment of the present disclosure will be described in detail below with reference to the accompanying drawings.

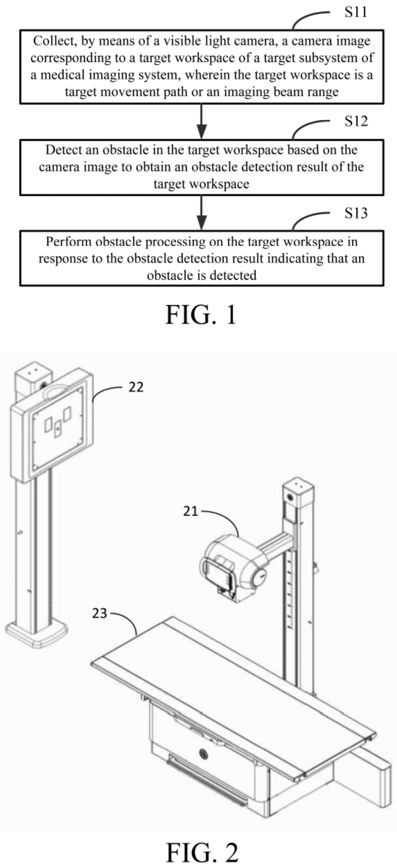

FIG. 1 shows a flowchart of the obstacle processing method for a medical imaging system provided in an embodiment of the present disclosure. In a possible implementation, an executing entity of the obstacle processing method for a medical imaging system may be an obstacle processing apparatus for a medical imaging system, for example, the obstacle processing method for a medical imaging system may be performed by a terminal device, a server, or other electronic devices. The terminal device may be a user equipment (UE), a mobile device, a user terminal, a terminal, or a personal digital assistant (PDA), a handheld device or a computing device, or the like. In some possible implementations, the obstacle processing method for a medical imaging system may be implemented by a processor by invoking computer-readable instructions stored in a memory. As shown in FIG. 1, the obstacle processing method for a medical imaging system includes step S11 to step S13.

In step S11, a camera image corresponding to a target workspace of a target subsystem of a medical imaging system is collected by means of a visible light camera, where the target workspace is a target movement path or an imaging beam range.

In step S12, an obstacle in the target workspace is detected based on the camera image to obtain an obstacle detection result of the target workspace.

In step S13, obstacle processing is performed on the target workspace in response to the obstacle detection result indicating that an obstacle is detected.

The medical imaging system may refer to a collection of devices for collecting, processing, storing, transmitting, and displaying images of internal structures of a human body. The medical imaging system plays a crucial role in medical diagnosis, treatment planning, disease monitoring, and research. Medical imaging technology can help doctors to observe details of the interior of the human body without invasive surgery.

The medical imaging system may include various types, such as an X-ray imaging system, a computed tomography (CT) scanning system, a magnetic resonance imaging (MRI) system, an ultrasound imaging system, a nuclear medicine imaging system, etc.

In the embodiments of the present disclosure, the target subsystem of the medical imaging system may be a subsystem capable of automatically moving in the medical imaging system, or a subsystem for emitting an imaging beam in the medical imaging system. The imaging beam may represent various types of energy beams used to generate images in the medical imaging system. For example, the imaging beam may include an X-ray beam used in X-ray imaging and CT scanning, an ultrasonic beam used in ultrasonic imaging, a radio frequency pulse used in magnetic resonance imaging, etc. For example, in a CT system, the imaging beam range may refer to a region covered by a spiral or cone beam emitted when the X-ray source rotates. In an MRI system, the imaging beam range may refer to a region covered by a radio frequency signal. In an ultrasonic imaging system, the imaging beam range may refer to a region covered by an ultrasonic beam; and so on and so forth.

In a possible implementation, the medical imaging system is an X-ray imaging system; and the target subsystem includes at least one of: an X-ray emission subsystem, an X-ray detection subsystem, and a patient support subsystem.

FIG. 2 shows a schematic view of the X-ray imaging system. In FIG. 2, the X-ray imaging system adopts a double-column structure. FIG. 2 shows an X-ray emission subsystem 21, an X-ray detection subsystem 22, and a patient support subsystem 23 in the X-ray imaging system. The X-ray emission subsystem 21 may include an X-ray emitter and a column for mounting the X-ray emitter, the X-ray detection subsystem 22 may include an X-ray detector and a chest stand for mounting the X-ray detector, and the patient support subsystem 23 may include an examination table. The movement of the X-ray emission subsystem includes: the column can drive the X-ray emitter to move in a transverse direction (a length direction of the examination table), the X-ray emitter can move relative to the column in a vertical direction or move along a longitudinal direction (longitudinal direction of the examination table), and the X-ray emitter can rotate relative to the column.

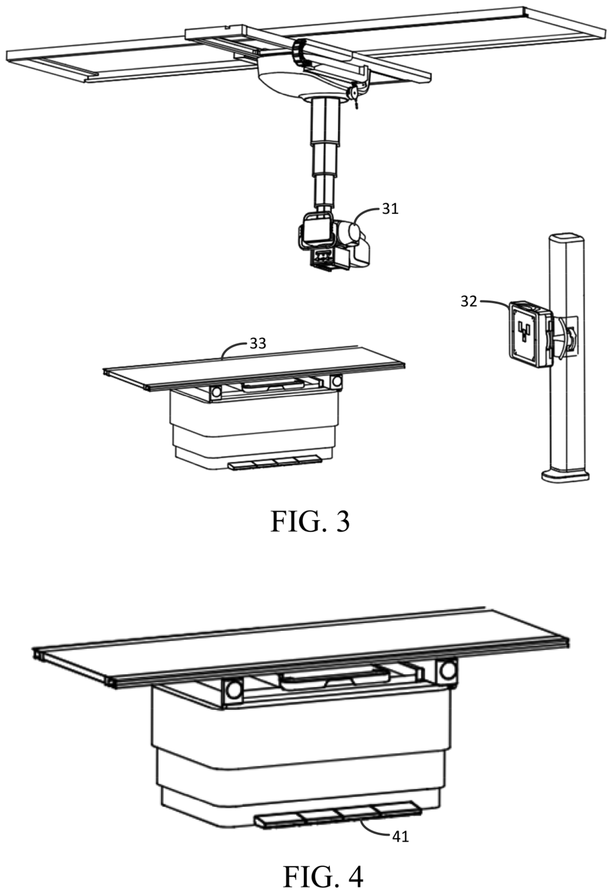

FIG. 3 shows another schematic view of the X-ray imaging system. FIG. 3 shows an X-ray emission subsystem 31, an X-ray detection subsystem 32, and a patient support subsystem 33 in the X-ray imaging system. The X-ray emission subsystem 31 adopts a ceiling-suspended structure, and the ceiling-suspended structure includes guide rails, a telescopic cylinder, a tube assembly, a tube control apparatus, etc. The guide rails are mounted on the ceiling, and the guide rails include a set of guide rails that are vertically mounted. The telescopic cylinder is mounted on the ceiling-suspended structure. The tube assembly is mounted on the telescopic cylinder, and an X-ray source and a collimator are provided in the tube assembly. The X-ray source is capable of emitting an X-ray beam, and the collimator is typically mounted below the X-ray source. The collimator is configured to confine and shape the X-ray beam to ensure that X-rays only irradiate a particular region while reducing unwanted radiation to surrounding tissues and organs. The tube control apparatus is mounted on the tube assembly, and includes user interfaces such as a display screen and a control button so as to be used to perform pre-capturing preparations, such as patient selection, protocol selection, positioning. The movement of the X-ray emission subsystem includes: the vertically mounted guide rails can drive the tube assembly to move in transverse and longitudinal directions, the telescopic cylinder can drive the tube assembly to move in the vertical direction, the tube assembly can rotate about the vertical direction, and the tube assembly can rotate about a direction perpendicular to a screen plane of the tube control apparatus.

The X-ray emission subsystem is a core component of the X-ray imaging system and is responsible for generating an X-ray beam. The X-ray emission subsystem may include a vacuum tube including a cathode (emitting electrons) and an anode (target material, typically tungsten). Electrons are emitted from the cathode to the anode at a high voltage to generate X-rays. The X-rays subsequently pass through the patient's body to form an image.

The X-ray detection subsystem may be configured to receive X-rays that pass through the patient's body and convert the X-rays into digital signals such that a computer can process the digital signals and generate images. In a digital X-ray imaging system, the X-ray detection subsystem may include a flat panel detector (FPD) which can directly convert X-rays into digital images without conventional films.

The movement of the X-ray detection subsystem may include: the chest stand can drive the X-ray detector to move in a transverse direction (length direction of the examination table), the X-ray detector can move relative to the chest stand in a vertical direction or move along a longitudinal direction (longitudinal direction of the examination table), and the X-ray detector can rotate relative to the chest stand.

The patient support subsystem may refer to a subsystem for a patient to lie down during an X-ray examination. The patient support subsystem may perform automatic movement and position adjustment to ensure that the patient's body part is correctly aligned with the X-ray beam and the X-ray detector. This mobility is essential for performing X-ray imaging from different angles and of different body sites.

FIG. 4 shows a schematic view of the patient support subsystem. As shown in FIG. 4, the patient support subsystem may include a foot pedal 41. A user may control the vertical movement of the tabletop of the examination table by operating the foot pedals of the patient support subsystem. When the user presses the foot pedal, a lifting mechanism of the examination table may be triggered, so that the tabletop may be raised or lowered to adjust the position of the patient to a height suitable for X-ray examination. When it is detected that the user releases the foot pedal, a foot pedal release signal may be generated, indicating that the user has completed the operation of the foot pedal. The foot pedal release signal may be configured to stop movement of the tabletop, or to confirm that the tabletop has reached a desired position. In some possible implementations, releasing the foot pedal may also trigger other functions, such as locking the position of the tabletop to ensure that the patient remains stable during the X-ray examination.

In a possible implementation, in a case where the target subsystem is a subsystem that can automatically move in the medical imaging system, the target workspace of the target subsystem may be a target movement path of the target subsystem. In this implementation, the target subsystem may be a component with automatic mobility in the medical imaging system. The target subsystem can move on a predetermined path (i.e., a target movement path) to perform a specific imaging task or adjust the position of the patient. The target workspace may refer to a spatial region involved in the movement process of the target subsystem. For example, when an X-ray examination is performed on the chest, the X-ray emission subsystem may be moved vertically downward to a position horizontal to the chest of the patient to image the chest from above. The target movement path of the X-ray detection subsystem may be opposite to the target movement path of the X-ray emission subsystem, or may rotate about the patient to capture images from different angles. The patient support subsystem may be moved vertically, horizontally, or tilted to adjust the position of the patient such that the patient is aligned with the X-ray emission subsystem and the X-ray detection subsystem.

In another possible implementation, in a case where the target subsystem is a subsystem configured to emit an imaging beam in the medical imaging system, the target workspace of the target subsystem may be a space region covered by the imaging beam emitted by the target subsystem, that is, an imaging beam range.

In a possible implementation, in a case where the medical imaging system is an X-ray imaging system, the imaging beam range may also be referred to as an X-ray fan beam range, or the like, which is not limited herein. In X-ray imaging, the imaging beam range may be determined according to a collimation region. The collimation region may refer to an irradiated region of an X-ray beam that is precisely defined by using the collimator. Before the X-ray imaging is performed, the collimation region may be set by a user e.g., a technician or medical personnel. The user may adjust the position and angle of the collimator according to the specific needs of the examination to define the path and range of the X-ray beam.

In the embodiments of the present disclosure, the camera image corresponding to the target workspace of the target subsystem of the medical imaging system may be collected by means of at least one visible light camera. The visible light camera may represent a device that captures an image using light in a visible spectrum range. The visible light camera may be configured to record and analyze light that can be perceived by the human eye, that is, light having a wavelength between about 400 nm (purple light) and 700 nm (red light). The camera image may refer to an image corresponding to the target workspace of the target subsystem captured by the visible light camera.

The visible light camera may be mounted at a fixed position in a scanning room, e.g., on a wall of the scanning room, or mounted on the medical imaging system, e.g., on a ceiling-suspended structure, specifically, on a side edge of a collimator, or the like, or fixed in any other manner. In some embodiments, the camera image is not limited to a single optical image, but may also include a dynamic real-time video stream, i.e., a series of real-time optical images.

In a possible implementation, the camera image corresponding to the target workspace of the target subsystem of the medical imaging system may be collected by means of only one visible light camera. By adopting this implementation, the cost of obstacle processing for the medical imaging system can be reduced.

In another possible implementation, the camera images corresponding to the target workspace of the target subsystem of the medical imaging system may be collected by means of at least two visible light cameras. In this implementation, the at least two visible light cameras may form a camera group or a camera platform.

In a possible implementation, the collection, by means of a visible light camera, of a camera image corresponding to a target workspace of a target subsystem of a medical imaging system comprises: collecting, by means of the visible light camera, a panoramic camera image corresponding to the medical imaging system, wherein the panoramic camera image covers the target workspace of the target subsystem of the medical imaging system.

In this implementation, the panoramic camera image corresponding to the medical imaging system may cover the entire medical imaging system, including the target workspace of the target subsystem, that is, a space where the target subsystem is located in the entire operating process. The panoramic camera image may assist in monitoring and recording the position, state and surrounding environment of the target subsystem, and ensure the safety and accuracy of the target subsystem in the operating process.

In this implementation, the panoramic camera image collected by the visible light camera provides comprehensive visual feedback for the medical imaging system, so that the operation of the target subsystem in the target workspace of the target subsystem can be controlled and managed more effectively.

In a possible implementation, the camera image includes RGB color information and depth information.

In this implementation, the camera image not only records color information of a scene, but also includes depth or distance information of each pixel relative to the camera. As one example of this implementation, the visible light camera may be an RGB-D camera, where "RGB" represents three color channels, i.e., red, green, and blue color channels, and "D" represents a depth channel.

The RGB color information refers to color data of each pixel point in the camera image, and is represented by different intensity combinations of the red, green, and blue color channels. The RGB color information enables the visible light camera to capture color details in the scene, thereby generating a color image. The depth information records a physical distance between each pixel in the scene and the visible light camera. In the RGB-D camera, the depth information may be obtained by a variety of techniques, such as structured light, Time of Flight (ToF), or stereo vision. A depth map may be represented in the form of a two-dimensional matrix in which each pixel value corresponds to an actual distance, rather than brightness or color information. The depth information enables the camera to perceive a three-dimensional structure of a scene where the medical imaging system is located, which provides additional dimensions for obstacle detection and helps to improve the accuracy of obstacle detection.

In another possible implementation, the camera image includes RGB color information. In this implementation, the camera image may include only the RGB color information, and does not include the depth information.

In a possible implementation, the method further comprises: in response to the presence of an occluding object in the camera image, controlling the visible light camera to move, and re-collecting the camera image by means of the visible light camera.

In this implementation, if an occluding object is detected in the camera image, that is, in the camera image, an object or personnel occludes the target workspace of the target subsystem, resulting in the camera image being unable to provide complete visual information of the target workspace, the visible light camera may be controlled to move, so as to change the position or angle of the visible light camera. Controlling the visible light camera to move is intended to circumvent or avoid the occluding object, so as to obtain an occlusion-free camera image.

After the visible light camera moves, the camera image may be captured again using the visible light camera. Since the position or angle of the visible light camera has been changed, it is expected that a camera image without occluding object interference can be obtained, so that the operating state and surrounding environment of the target subsystem can be monitored and analyzed more accurately.

This implementation assists the medical imaging system in continuously obtaining complete and accurate visual information during operation, which is crucial for safe operation and effective diagnosis of the medical imaging system. By dynamically adjusting the position of the visible light camera to avoid the occluding object, the implementation can adapt to complex and dynamic working environments, enhancing quality and reliability of the camera image.

In another possible implementation, the method further comprises: in response to the presence of an occluding object in the camera image, issuing an occlusion prompt message; and in response to removal of the occluding object, re-collecting the camera image by means of the visible light camera.

In this implementation, if the occluding object is detected in the camera image, the occlusion prompt message may be automatically issued. The occlusion prompt message may be a visual warning, an audio alarm, or another type of indication, used to notify an operator of the presence of the occluding object. After receiving the prompt, the operator may take measures to remove the occluding object.

In yet another possible implementation, in a case where an occluding object exists in the camera image, obstacle detection may be performed on the target workspace based on an unoccluded image region in the camera image.

In the embodiments of the present disclosure, after the camera image corresponding to the target workspace of the target subsystem of the medical imaging system is collected by means of the visible light camera, an obstacle in the target workspace may be detected based on the camera image to obtain an obstacle detection result of the target workspace. In a case where the target workspace is the target movement path, it may be determined that an obstacle is detected on the target movement path in response to a distance between any object (object or personnel) and the target movement path being less than or equal to a distance threshold. In a case where the target workspace is the imaging beam range, it may be determined that an obstacle, i.e., a foreign object, is detected within the imaging beam range in response to detecting any object within the imaging beam range. The obstacle detection result of the target workspace may include that an obstacle is detected or an obstacle is not detected. In a case where the obstacle detection result of the target workspace includes that an obstacle is detected, the obstacle detection result of the target workspace may further include at least part of information such as a position, a type, and a confidence of the obstacle.

The position of the obstacle may be represented in the form of a bounding box, two dimensional coordinates, three dimensional coordinates, or the like, which is not limited herein.

The type of the obstacle may be represented in the form of a classification label, an integer encoding, or the like, which is not limited herein. For example, the type of the obstacle may be represented by a predefined classification label, such as "patient", "medical personnel", "medical device", "furniture". For another example, the type of the obstacle may be represented using an integer encoding, for example, 0 representing "patient", 1 representing "medical personnel", 2 representing "medical device", or the like.

The confidence is an indicator for measuring the certainty of the prediction result of the model. In the embodiments of the present disclosure, the confidence may represent the model's certainty level as to whether a certain detected object is an obstacle. The confidence may be a numerical value between 0 and 1, where a higher value indicates that the model is more certain that the object is an obstacle. In a machine learning model, the confidence may be calculated in various ways. For example, in classification issues, the model may output a probability distribution, and the confidence may be a probability of a category corresponding to a maximum value in the distribution.

In a possible implementation, the obstacle includes at least one of the following types: other subsystems of the medical imaging system, a patient, a technician or medical personnel, and other objects in a space where the medical imaging system is located.

As one example of this implementation, the obstacle may include other subsystems in the medical imaging system other than the target subsystem. For example, in a case where the target subsystem is an X-ray emission subsystem, the patient support subsystem may also become an obstacle of the X-ray emission subsystem on the target movement path.

As one example of this implementation, the obstacle may include a patient. Patients are one of the most important considerations in medical imaging systems. For example, during X-ray imaging or other examinations, it is necessary to ensure the position of the patient is safe and ensure that the patient does not collide with the moving subsystem. Additionally, possible movement of the patient may also be taken into account to avoid accidents during imaging.

As one example of this implementation, the obstacle may include a technician or medical personnel. The technicians or medical personnel are professionals who may be located on the target movement path or within the imaging beam range during operation of the medical imaging system. They may enter the target workspace when adjusting the position of the patient or operating devices, or performing other medical operations. Therefore, in this example, by detecting the positions of the technician or medical personnel and stopping or adjusting movement of the target subsystem as necessary, their safety is safeguarded.

As one example of this implementation, the obstacle may include other objects in a space where the medical imaging system is located. For example, the other objects in the space where the medical imaging system is located may include a patient footstool, a wheelchair, an infusion stand, a medical cart, other medical instruments, or the like. These objects may unintentionally enter the target movement path or imaging beam range of the target subsystem, thereby causing safety hazards or affecting imaging quality.

In a possible implementation, the positions of various subsystems (including the target subsystem) in the medical imaging system may be determined based on the camera image. In this implementation, the positions of the subsystems may be obtained by capturing the three-dimensional coordinates or two-dimensional coordinates of the subsystems in space by means of the visible light camera.

In another possible implementation, the positions of the subsystems may be determined according to hardware feedback of the subsystems of the medical imaging system. In this implementation, sensors or another positioning devices may be installed on the subsystems to directly provide position data of the subsystems. The position data provided by the sensors or other positioning devices may be the absolute positions (e.g., absolute coordinates) or relative positions (e.g., positions in a robotic arm or C-arm) of the subsystem.

In another possible implementation, the positions of the subsystems may be determined by combining the camera image and the hardware feedback of the subsystems.

In a possible implementation, the detection of an obstacle in the target workspace based on the camera image to obtain an obstacle detection result of the target workspace comprises: inputting the camera image into a pre-trained obstacle detection model, and detecting, by means of the obstacle detection model, the obstacle in the target workspace; and determining the obstacle detection result of the target workspace based on a detection result of the obstacle detection model.

In this implementation, the obstacle detection model may be a machine learning model. For example, the obstacle detection model may be a YOLOv5 model, a Faster R-CNN model, an SSD model, or the like, which is not limited herein.

In the training stage of the obstacle detection model, the obstacle detection model may be trained using a training image set including a large number of training images. The training image may be annotated with the bounding box coordinates of an object and the type of the object. The training images in the training image set may include various variations, so that the obstacle detection model can generalize and detect obstacles having different sizes, from different angles, and under different environments. That is, the training image set may include images captured from different angles, images under different lighting conditions, images in different backgrounds and scenes, etc., to cover various situations that may be encountered in practical applications. By training the obstacle detection model using the labeled training images, the obstacle detection model can learn how to recognize and locate a particular object in an image. During training, the obstacle detection model may continuously adjust its internal parameters to minimize the difference between a predicted bounding box and a true bounding box, thereby improving the accuracy of obstacle detection.

The output of the obstacle detection model may include position information of the detected obstacle. For example, the position information of the obstacle may be represented by means of a bounding box. The bounding box can define the position and size of the obstacle within the image in the form of coordinates.

In a possible implementation, the determination of the obstacle detection result of the target workspace based on a detection result of the obstacle detection model includes: recognizing, by means of a pre-trained position recognition model, a position of an object of a preset type in the target workspace to obtain a position recognition result of the object of the preset type; and determining the obstacle detection result of the target workspace based on the detection result of the obstacle detection model and the position recognition result of the object of the preset type.

In this implementation, the position recognition model may be pre-trained, and the position recognition model may be used to recognize and locate an object of a specific type (e.g., personnel, a table top, etc.) in the target workspace. That is, the position recognition model may recognize objects of a preset type and may provide their position information in images.

As one example of this implementation, the pre-trained position recognition model may recognize the position of the object of the preset type in the target workspace based on the camera image, to obtain the position recognition result of the object of the preset type.

As one example of this implementation, in response to the presence of an occluding object in the camera image, the position of the object of the preset type in the target workspace may be recognized by means of the pre-trained position recognition model to obtain the position recognition result of the object of the preset type.

In this implementation, the output of the obstacle detection model and the output (the position information of the object of the preset type) of the position recognition model may be combined to determine the final detection result of the obstacle in the target workspace. Such combination can provide more comprehensive situational awareness, helping to more accurately detect obstacles in the target workspace.

In a possible implementation, the position recognition model recognition includes a tabletop position recognition model and/or a personnel position recognition model, and the preset type comprises tabletop and/or personnel.

As one example of this implementation, the position recognition model recognition includes a tabletop position recognition model, and the preset type includes tabletop. In this example, a position recognition result of a tabletop output by the tabletop position recognition model may include the height of the tabletop. By recognizing the position of the tabletop, it is possible to assist in determining whether the table is in the target workspace, that is, it is possible to assist in determining whether the tabletop belongs to an obstacle in the target workspace.

In one example, the tabletop position recognition model may determine the position of the tabletop (e.g., an X-ray examination table) using specific recognition points (e.g., two-dimensional codes, reflective marks, or other recognizable feature points). The recognition points are pre-placed on the tabletop, the tabletop position recognition model may detect the recognition points using the image recognition technology and calculate their positions relative to the visible light camera, thereby inferring the position of the tabletop. Two or more recognition points may be provided on the tabletop. For example, the recognition points may be provided on both sides of the tabletop. By setting two or more recognition points on the tabletop, the accuracy of tabletop position recognition is improved. In a case where the recognition point is occluded by personnel (e.g., a technician or medical personnel standing in front of the point), the position of the tabletop may be recalculated after waiting for the personnel to leave. In a case where the position of the tabletop cannot be determined based on the recognition points, it may be reported that the position of the tabletop is unknown.

As another example of this implementation, the position recognition model recognition includes a personnel position recognition model, and the preset type includes personnel. By recognizing the position of the personnel, it is possible to assist in determining whether there is a person in the target workspace, that is, it is possible to assist in determining whether there is a person belonging to an obstacle in the target workspace. In one example, the personnel position recognition model may be configured to recognize whether the patient is lying down on the examination table.

In a possible implementation, obstacle detection may be performed solely using the obstacle detection model, without relying on the position recognition model. In this implementation, the types of obstacles detected by the obstacle detection model may include tabletops and personnel, and may further include subsystems, footstools, wheelchairs, infusion stands, medical carts, etc. Furthermore, in this implementation, the detection result of the obstacle detection model may be directly determined as the obstacle detection result of the target workspace.

In a possible implementation, in a case where the target workspace is the target movement path and the obstacle detection result of the target workspace is that no obstacle is detected, the target subsystem may be controlled to move along the target movement path.

In a possible implementation, in a case where the target workspace is the imaging beam range and the obstacle detection result of the target workspace is that no obstacle is detected, the medical imaging system may be allowed to be controlled to perform medical image collection, for example, to perform X-ray exposure.

In a possible implementation, the performance of obstacle processing on the target workspace comprises: displaying a bounding box surrounding the obstacle in a video stream of the visible light camera according to coordinates of the obstacle.

In this implementation, after the obstacle is detected, the coordinates of the obstacle in the camera image may be obtained. The coordinates define the position and boundaries of the obstacle in the camera image. A bounding box may be drawn in the video stream of the visible light camera to surround each detected obstacle according to the coordinates of the obstacle. The bounding box is a visual marker used to highlight a particular object in an image or video frame. The bounding box is overlaid on the video stream to display the position of the obstacle in real time.

By adopting this implementation, the operator can intuitively see the exact position of the obstacle and make corresponding operation decisions accordingly. By displaying the bounding box of the obstacle in the video stream, real-time visual feedback is provided, helping the operator to monitor the status of the target workspace and take measures in time to avoid potential collisions or artifacts.

As one example of this implementation, the confidence that the object in the bounding box belongs to an obstacle may also be displayed in the video stream of the visible light camera. For example, in the video stream of a visible light camera, the confidence may be displayed as a decimal, a percentage value, or a bar graph next to or inside each bounding box. Such visualization can help the operator to quickly evaluate the reliability of the obstacle detection result, so that the operator can determine whether to take action according to the confidence. For example, if the confidence is high, the operator may immediately take measures to remove the obstacle; and if the confidence is low, the operator may choose to perform further observation or manual confirmation.

As one example of this implementation, the type of the obstacle to which the object in the bounding box belongs may also be displayed in the video stream of the visible light camera. For example, next to or inside each bounding box, the type label of the obstacle may be directly displayed. For example, if the detected obstacle is a "patient", then a word "patient" may be displayed next to the bounding box.

As one example of this implementation, in the medical imaging system, the video stream of the visible light camera may be displayed by means of a graphical user interface (GUI), and the bounding box surrounding the obstacle is displayed in the video stream of the visible light camera. The GUI may be integrated on a main controller of a control room, or may be directly embedded into a display screen of a tube control apparatus of the X-ray emission subsystem, so that the operator can intuitively monitor and analyze the status of the target workspace and make corresponding operation decisions in time.

In a possible implementation, the performance of obstacle processing on the target workspace comprises: outputting obstacle warning information.

The obstacle warning information may take a variety of forms, including but not limited to a visual prompt (e.g., a flashing icon or a textual message on a screen), an auditory prompt (e.g., a beep or a voice prompt), or other forms of notification, with the purpose of immediately reminding the operator of a potential collision risk or imaging obstacle.

This implementation can improve the safety and reliability of the medical imaging system during operation. By providing the obstacle warning information in time, the technician or medical personnel can take measures quickly to prevent the medical imaging system from colliding with the patient or other obstacles, thereby reducing the risk of equipment damage, protecting the patient from injury, and ensuring the continuity and imaging quality of the imaging process. Such proactive warning mechanism also helps to reduce medical accidents caused by operation errors, and improve the situational awareness of medical personnel regarding their surroundings, thereby optimizing the working efficiency and diagnostic accuracy of the entire medical imaging system.

In a possible implementation, in a case where the target workspace is the target movement path, the performance of obstacle processing on the target workspace comprises: controlling the target subsystem to pause movement in response to a distance between the target subsystem and the obstacle being less than or equal to a preset distance, and controlling the target subsystem to continue moving along the target movement path in response to the obstacle being removed.

In this implementation, when the target subsystem (e.g., the X-ray emission subsystem or the X-ray detection subsystem) moves on the predetermined movement path (i.e., the target movement path), the distance between the target subsystem and the obstacle may be monitored in real time. If the distance between the target subsystem and the obstacle is less than or equal to a preset safety distance threshold (i.e., the preset distance), the movement of the target subsystem may be automatically paused to avoid a potential collision. After the obstacle is removed, the movement of the target subsystem may be automatically resumed, allowing it to continue advancing along the target movement path.

This implementation can improve the safety and reliability of the medical imaging system during operation. By automatically detecting the obstacle and controlling the movement of the target subsystem, it is possible to effectively prevent the target subsystem from accidentally colliding with the patient or other obstacles, reduce the risk of equipment damage, protect the patient from harm, and ensure the safety of medical personnel. Furthermore, such automatic control mechanism further improves the level of intelligence of the medical imaging system, reduces the workload of the operator, and makes the medical imaging process smoother and more efficient.

In a possible implementation, in a case where the target workspace is the target movement path, the performing obstacle processing on the target workspace comprises: generating an alternative movement path for the target movement path, and controlling the target subsystem to move along the alternative movement path, wherein no obstacle exists on the alternative movement path.

In this implementation, when the target subsystem of the medical imaging system encounters an obstacle on its target movement path, the target subsystem can not only detect the obstacle, but also intelligently generate a new alternative movement path that avoids the obstacle. Then, the movement of the target subsystem may be automatically adjusted such that the target subsystem continues to perform its task along the new alternative path, thereby ensuring that necessary imaging operations can be completed without avoiding collision with the obstacle.

This implementation can improve the adaptability and flexibility of the medical imaging system in complex environments. By automatically avoiding obstacles and re-planning the path, the continuity and efficiency of the imaging process can be ensured, and imaging interruptions caused by obstacles can be reduced. This not only improves the imaging quality and reduces the need for repeated imaging but also lowers the risk of radiation exposure for both patients and medical personnel, and also alleviates the decision-making burden of operators in complex situations, thereby improving overall work efficiency and safety.

In a possible implementation, in a case where the target workspace is the target movement path, the performing obstacle processing on the target workspace comprises: controlling the obstacle to move so as to avoid the target movement path in response to the obstacle being a movable subsystem of the medical imaging system and the obstacle being in a movement-allowable state.

In this implementation, if the obstacles on the target movement path are movable subsystems (e.g., the movable X-ray detection subsystem or the patient support subsystem) of the medical imaging system, and the movable subsystems are in a movement-allowable state, the obstacles may be automatically controlled to move to another position, to avoid the target movement path. In this implementation, both the obstacles can be detected, and the positions of the obstacles can be actively adjusted, thereby removing the obstacles on the moving path, and ensuring that the target subsystem (e.g., the X-ray emission subsystem) can move smoothly without pausing or changing the predetermined workflow.

This implementation can enhance the autonomy and flexibility of the medical imaging system in complex environments. By automatically moving obstacles to clear the path, imaging delays caused by the obstacles can be reduced, thereby improving the imaging efficiency; and the need for operators to manually adjust the device can be reduced, thereby reducing the operation complexity. Furthermore, such automated obstacle processing mechanism can also reduce risks arising from manual operations, thereby enhancing the safety of patients and medical personnel, and ensuring that the medical imaging system can perform imaging operations more smoothly and safely.

In a possible implementation, in a case where the target workspace is the imaging beam range, the performing obstacle processing on the target workspace comprises: controlling the medical imaging system to pause medical image collection; and in response to the obstacle being removed, allowing control of the medical imaging system to continue the medical image collection.

In this implementation, when the target workspace of the medical imaging system is the imaging beam range, it may be monitored whether an obstacle exists in the imaging beam range. If an obstacle is detected in the imaging beam range, the ongoing medical image collection process may be automatically paused to avoid imaging artifacts. After the obstacle is removed, the process may be restarted to allow the user to continue image collection. Such processing method ensures that the imaging quality is not affected by obstacles and the patient is protected from potential injury.

This implementation can ensure that the imaging environment is clear and unobstructed when medical image collection is performed, thereby improving the quality and accuracy of imaging. By prompting or automatically removing obstacles, repeated exposure caused by the obstacles can be reduced, thereby saving time and resources, and reducing the radiation exposure risk for patients and medical personnel. In addition, this implementation further improves the level of intelligence of the medical imaging system, making the operation safer and more efficient.

In a possible implementation, data may be exchanged between different components (e.g., a wall stand control platform and a position algorithm platform) of the medical imaging system by means of an atlas data bus. The wall stand control platform may receive data from the position algorithm platform, and control the movement and positions of wall stands for X-ray emitters and detectors according to the data. The position algorithm platform is a software platform for processing position-related algorithms, and is configured to process position-related algorithms, such as obstacle detection, location tracking, and path planning. The position algorithm platform may utilize data collected from the visible light camera and other sensors to compute position information of the obstacle, and send a command to the wall stand control platform by means of CAN-BUS to control the position of the wall stand. The wall stand may refer to a support or carrying apparatus for carrying X-ray emitters and X-ray detectors. In the X-ray imaging system, the wall stands may take different forms, with the primary purpose of supporting and moving the X-ray emitter and the X-ray detector, so as to facilitate imaging of a patient at different angles and positions. For example, the wall stand may include a column and a chest stand as shown in FIG. 2. For another example, the wall stand may include a ceiling-suspended structure and a chest stand as shown in FIG. 3.

It can be understood that all the foregoing method embodiments mentioned in the present disclosure may be combined with each other to form a combined embodiment without departing from the principle or logic, which will not be described in detail in the present disclosure due to limited space. It can be understood by those skilled in the art that, in the above methods described in the detailed embodiments, the specific order in which each step is performed should be determined by the function and possible inherent logic thereof.

In addition, the present disclosure further provides an obstacle processing apparatus for a medical imaging system, an electronic device, a computer-readable storage medium, and a computer program product, all of which can be used to implement any one of the obstacle processing methods for a medical imaging system provided by the present disclosure. For corresponding technical solutions and technical effects, reference may be made to corresponding descriptions in the method sections, and details will not be described herein again.

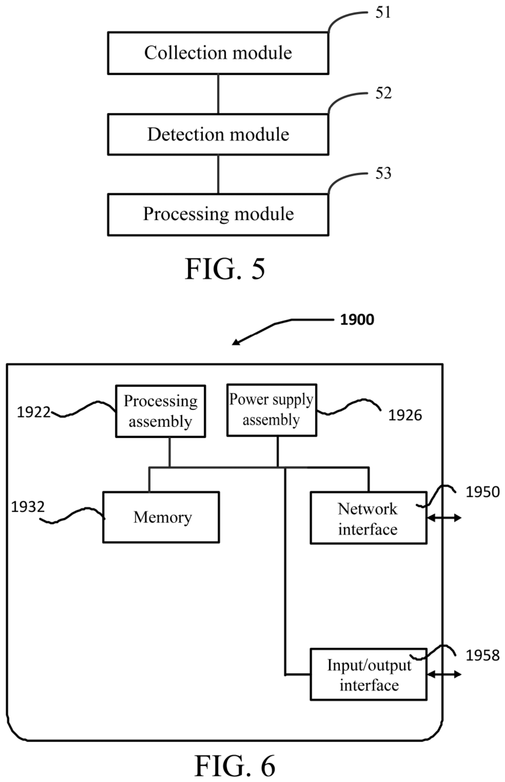

FIG. 5 shows a block diagram of an obstacle processing apparatus for a medical imaging system provided in an embodiment of the present disclosure. As shown in FIG. 5, the obstacle processing apparatus for a medical imaging system includes:
a collection module 51, configured to collect, by means of a visible light camera, a camera image corresponding to a target workspace of a target subsystem of a medical imaging system, wherein the target workspace is a target movement path or an imaging beam range;
a detection module 52, configured to detect an obstacle in the target workspace based on the camera image to obtain an obstacle detection result of the target workspace; and
a processing module 53, configured to perform obstacle processing on the target workspace in response to the obstacle detection result indicating that an obstacle is detected.

In a possible implementation, the collection module 51 is configured to:
collecting, by means of the visible light camera, a panoramic camera image corresponding to the medical imaging system, wherein the panoramic camera image covers the target workspace of the target subsystem of the medical imaging system.

In a possible implementation, the camera image includes:
RGB color information and depth information;
   or,
RGB color information.

In a possible implementation, the apparatus further includes:
a moving module, configured to, in response to the presence of an occluding object in the camera image, control the visible light camera to move, and re-collect the camera image by means of the visible light camera.

In a possible implementation, the detection module 52 is configured to:
inputting the camera image into a pre-trained obstacle detection model, and detecting, by means of the obstacle detection model, the obstacle in the target workspace; and
determine the obstacle detection result of the target workspace based on a detection result of the obstacle detection model.

In a possible implementation, the detection module 52 is configured to:
recognizing, by means of a pre-trained position recognition model, a position of an object of a preset type in the target workspace to obtain a position recognition result of the object of the preset type; and
determine the obstacle detection result of the target workspace based on the detection result of the obstacle detection model and the position recognition result of the object of the preset type.

In a possible implementation, the position recognition model recognition includes a tabletop position recognition model and/or a personnel position recognition model, and the preset type comprises tabletop and/or personnel.

In a possible implementation, the processing module 53 is configured to:
display a bounding box surrounding the obstacle in a video stream of the visible light camera according to coordinates of the obstacle.

In a possible implementation, the processing module 53 is configured to:
output obstacle warning information.

In a possible implementation, in a case where the target workspace is the target movement path, the processing module 53 is configured to:
control the target subsystem to pause movement in response to a distance between the target subsystem and the obstacle being less than or equal to a preset distance, and control the target subsystem to continue moving along the target movement path in response to the obstacle being removed;
   or,
generate an alternative movement path for the target movement path, and control the target subsystem to move along the alternative movement path, wherein no obstacle exists on the alternative movement path;
   or,
control the obstacle to move so as to avoid the target movement path in response to the obstacle being a movable subsystem of the medical imaging system and the obstacle being in a movement-allowable state.

In a possible implementation, in a case where the target workspace is the imaging beam range, the processing module 53 is configured to:
control the medical imaging system to pause medical image collection; and
in response to the obstacle being removed, allow control of the medical imaging system to continue the medical image collection.

In a possible implementation, the medical imaging system is an X-ray imaging system; and
the target subsystem includes at least one of: an X-ray emission subsystem, an X-ray detection subsystem, and a patient support subsystem.

In a possible implementation, the obstacle includes at least one of the following types: other subsystems of the medical imaging system, a patient, a technician or medical personnel, and other objects in a space where the medical imaging system is located.

In some embodiments, the function of the apparatus provided in the embodiments of the present disclosure or the modules included in the apparatus may be configured to perform the method described in the foregoing method embodiments, and for specific implementation and technical effects thereof, reference may be made to the description of the foregoing method embodiments, which are not described herein again for brevity.

An embodiment of the present disclosure further provides a computer-readable storage medium, having computer program instructions stored thereon, wherein the computer program instructions, when executed by a processor, implement the above method. The computer-readable storage medium may be a non-volatile computer-readable storage medium or a volatile computer-readable storage medium.

An embodiment of the present disclosure further provides a computer program, including computer-readable code, wherein when the computer-readable code is run in an electronic device, a processor in the electronic device performs the above method.

An embodiment of the present disclosure further provides a computer program product, including computer-readable code or a non-volatile computer-readable storage medium bearing computer-readable code, wherein when the computer-readable code is run in an electronic device, a processor in the electronic device performs the above method.

An embodiment of the present disclosure further provides an electronic device, including: one or more processors; and a memory for storing executable instructions, wherein the one or more processors are configured to invoke the executable instructions stored by the memory to perform the above method.

The electronic device may be provided as a terminal, a server or a device in another form.

FIG. 6 shows a block diagram of an electronic device 1900 provided in an embodiment of the present disclosure. For example, the electronic device 1900 may be provided as a terminal or a server. Referring to FIG. 6, the electronic device 1900 includes a processing assembly 1922 that further includes one or more processors, and a memory resource represented by a memory 1932 for storing instructions that can be executed by the processing assembly 1922, such as an application. The application stored in the memory 1932 can include one or more modules each corresponding to a set of instructions. In addition, the processing assembly 1922 is configured to execute instructions to execute the above method.

The electronic device 1900 can further include a power supply assembly 1926 configured to perform power management of the electronic device 1900, a wired or wireless network interface 1950 configured to connect the electronic device 1900 to a network, and an input/output (I/O) interface 1958. The electronic device 1900 can operate on the basis of an operating system stored in the memory 1932, for example, a Microsoft server operating system (Windows Server^{™}), a graphical user interface-based operating system (Mac OS X^{™}) provided by Apple Inc., a multi-user multi-process computer operating system (Unix^{™}), a free and open source Unix-like operating system (Linux^{™}), an open source Unix-like operating system (FreeBSD^{™}) or the like.

In an exemplary embodiment, a non-volatile computer-readable storage medium is also provided, such as the memory 1932 including computer program instructions that are executable by the processing assembly 1922 of the electronic device 1900 to perform the above method.

The present disclosure may be a system, a method and/or a computer program product. The computer program product may include a computer-readable storage medium, having computer-readable program instructions thereon for causing a processor to implement various aspects of the present disclosure.

The computer-readable storage medium can be a tangible device that can hold and store instructions used by an instruction execution device. The computer-readable storage medium may be, for example, but is not limited to an electrical storage device, a magnetic storage device, an optical storage device, an electromagnetic storage device, a semiconductor memory device, or any suitable combination thereof. More specific examples (a non-exhaustive list) of the computer-readable storage medium include: a portable computer disk, a hard disk, a random access memory (RAM), a read only memory (ROM), a erasable programmable read only memory (EPROM or flash memory), a static random access memory (SRAM), a portable compact disc read-only memory (CD ROM), a digital versatile disk (DVD), a memory stick, a floppy disk, mechanical coding equipment, such as a punch card having instructions stored thereon or a structure of bumps within recessions, and any suitable combination thereof. The computer-readable storage medium used herein is not interpreted as transient signals themselves, such as radio waves or other freely propagated electromagnetic waves, electromagnetic waves propagated through a waveguide or other transmission media (e.g., light pulses passing through a fiber optic cable), or electrical signals transmitted through electric wires.

The computer-readable program instructions described herein may be downloaded from a computer-readable storage medium to various computing/processing devices or downloaded to an external computer or external storage device via a network such as the Internet, a local area network, a wide area network and/or a wireless network. The network may include copper transmission cables, fiber transmission, wireless transmission, routers, firewalls, switches, gateway computers, and/or edge servers. A network adapter card or a network interface in each computing/processing device receives computer-readable program instructions from the network and forwards the computer-readable program instructions, for storing them in a computer-readable storage medium in each computing/processing device.

Computer program instructions for executing the operations of the present disclosure can be assembly instructions, instruction set architecture (ISA) instructions, machine instructions, machine related instructions, microcode, firmware instructions, state setting data, or source code or object code written in any combination of one or more programming languages, the programming language including object oriented programming languages such as Smalltalk, C++ and the like, and conventional procedural programming languages such as the "C" language or similar programming languages. The computer-readable program instructions can be executed entirely or partly on a user computer, executed as a stand-alone software package, executed partly on a user computer and partly on a remote computer, or executed entirely on a remote computer or server. In the case of a remote computer, the remote computer may be connected to the user's computer through any kind of network, including a local area network (LAN) or a wide area network (WAN). Alternatively, it can be connected to an external computer (for example, using an Internet service provider to connect via the Internet). In some embodiments, an electronic circuit, for example, a programmable logic circuit, a field-programmable gate array (FPGA), or a programmable logic array (PLA), may execute the computer-readable program instructions by utilizing state information of the computer-readable program instructions to personalize the electronic circuit, in order to implement various aspects of the present disclosure.

The aspects of the present disclosure are described herein with reference to the flowcharts and/or block diagrams of the methods, apparatuses (systems), and computer program products according to the embodiments of the present disclosure. It should be understood that each block of the flowcharts and/or block diagrams and combinations of various blocks in the flowcharts and/or block diagrams can be implemented by computer-readable program instructions.

These computer-readable program instructions may be provided to a processor of a general-purpose computer, a special-purpose computer, or other programmable data processing apparatuses, to produce a machine, so that these instructions, when executed by the processor of the computer or other programmable data processing apparatuses, produce an apparatus for implementing the functions/actions specified in one or more blocks of the flowcharts and/or block diagrams. Also, these computer-readable program instructions may be stored in a computer-readable storage medium. These instructions cause a computer, a programmable data processing apparatus, and/or other devices to work in a specific manner. Thus, the computer-readable medium storing the instructions includes an artifact, including instructions that implement various aspects of the functions/actions specified in one or more the flowcharts and/or block diagrams.

The computer-readable program instructions may also be loaded onto a computer, other programmable data processing apparatuses, or other devices, such that the computer, other programmable data processing apparatuses or other devices execute a series of operational steps, to generate a computer-implemented process, such that the functions/actions specified in one or more of the flowcharts and/or block diagrams are implemented by the instructions executed on the computer, other programmable data processing apparatuses, or other devices.

The flowcharts and block diagrams in the accompanying drawings illustrate system architectures, functions, and operations of possible implementations of the system, method, and computer program product according to a plurality of embodiments of the present disclosure. In this regard, each block in the flowcharts or block diagrams may represent a portion of a module, program segment, or instruction that contains one or more executable instructions for implementing the specified logical functions. In some alternative implementations, the functions denoted in the blocks can also occur in a different order than that illustrated in the drawings. For example, two consecutive blocks can actually be executed substantially in parallel, and sometimes can also be executed in a reverse order, depending upon the functions involved. It should also be noted that each block of the block diagrams and/or flowcharts, and combinations of blocks in the block diagrams and/or flowcharts can be implemented in a dedicated hardware-based system that executes the specified function or action, or can be implemented by a combination of dedicated hardware and computer instructions.

The computer program product may be embodied in hardware, software, or a combination thereof. In an alternative embodiment, the computer program product is embodied as a computer storage medium, and in another alternative embodiment, the computer program product is embodied as a software product, such as a software development kit (SDK) or the like.

The above description of the various embodiments tends to emphasize differences between the various embodiments, and reference may be made therebetween for the same features or similarities thereof, which will not be described in detail herein for the sake of brevity.

If the technical solution of the embodiments of the present disclosure involves personal information, a product to which the technical solution of the embodiments of the present disclosure is applied has explicitly performed notification of a personal information processing rule before processing the personal information, and has acquired individual self-consent. If the technical solution of the embodiments of the present disclosure involves sensitive personal information, a product to which the technical solution of the embodiments of the present disclosure is applied has acquired individual consent before processing the sensitive personal information, and also satisfies the requirement of "explicit consent". For example, at a personal information collection apparatus such as a camera, a clear and obvious sign is provided to state that a personal information collection range has been entered, and personal information will be collected. If a person voluntarily enters the collection range, it is regarded as consent to collect personal information thereof. Alternatively, if notification of a personal information processing rule is performed on a personal information processing apparatus by using an obvious sign/information, personal authorization is acquired by means of pop-up information or by inviting the individual to upload personal information thereof. The personal information processing rule may include information such as a personal information handler, a personal information processing purpose, a processing method, and a type of personal information to be processed.

The embodiments of the present disclosure have been described above. The foregoing description is illustrative rather than limiting, and is not limited to the disclosed embodiments. Many modifications and variations are apparent to those of ordinary skill in the art without departing from the scope and spirit of the embodiments illustrated. The selection of terms used herein is intended to best explain the principles, practical applications, or improvements to the techniques in the market of the embodiments, or to enable others of ordinary skill in the art to understand the embodiments disclosed herein.

## Claims

1. An obstacle processing method for a medical imaging system, **characterized by** comprising:
collecting, by means of a visible light camera, a camera image corresponding to a target workspace of a target subsystem of a medical imaging system, wherein the target workspace is a target movement path or an imaging beam range;
detecting an obstacle in the target workspace based on the camera image to obtain an obstacle detection result of the target workspace; and
performing obstacle processing on the target workspace in response to the obstacle detection result indicating that an obstacle is detected.

2. The method according to claim 1, **characterized in that** the collection, by means of a visible light camera, of a camera image corresponding to a target workspace of a target subsystem of a medical imaging system comprises:
collecting, by means of the visible light camera, a panoramic camera image corresponding to the medical imaging system, wherein the panoramic camera image covers the target workspace of the target subsystem of the medical imaging system.

3. The method according to claim 1, **characterized in that** the camera image comprises:
RGB color information and depth information;
or,
RGB color information.

4. The method according to claim 1, **characterized by** further comprising:
in response to the presence of an occluding object in the camera image, controlling the visible light camera to move, and re-collecting the camera image by means of the visible light camera.

5. The method according to any one of claims 1 to 4, **characterized in that** the detection of an obstacle in the target workspace based on the camera image to obtain an obstacle detection result of the target workspace comprises:
inputting the camera image into a pre-trained obstacle detection model, and detecting, by means of the obstacle detection model, an obstacle in the target workspace; and
determining the obstacle detection result of the target workspace based on a detection result of the obstacle detection model.

6. The method according to claim 5, **characterized in that** the determination of the obstacle detection result of the target workspace based on a detection result of the obstacle detection model comprises:
recognizing, by means of a pre-trained position recognition model, a position of an object of a preset type in the target workspace to obtain a position recognition result of the object of the preset type; and
determining the obstacle detection result of the target workspace based on the detection result of the obstacle detection model and the position recognition result of the object of the preset type.

7. The method according to claim 6, **characterized in that** the position recognition model recognition comprises a tabletop position recognition model and/or a personnel position recognition model, and the preset type comprises tabletop and/or personnel.

8. The method according to any one of claims 1 to 4, **characterized in that** the performing obstacle processing on the target workspace comprises:
displaying a bounding box surrounding the obstacle in a video stream of the visible light camera according to coordinates of the obstacle.

9. The method according to any one of claims 1 to 4, **characterized in that** the performing obstacle processing on the target workspace comprises:
outputting obstacle warning information.

10. The method according to any one of claims 1 to 4, **characterized in that** in a case where the target workspace is the target movement path, the performing obstacle processing on the target workspace comprises:
controlling the target subsystem to pause movement in response to a distance between the target subsystem and the obstacle being less than or equal to a preset distance, and controlling the target subsystem to continue moving along the target movement path in response to the obstacle being removed;
or,
generating an alternative movement path for the target movement path, and controlling the target subsystem to move along the alternative movement path, wherein no obstacle exists on the alternative movement path;
or,
controlling the obstacle to move so as to avoid the target movement path in response to the obstacle being a movable subsystem of the medical imaging system and the obstacle being in a movement-allowable state.

11. The method according to any one of claims 1 to 4, **characterized in that** in a case where the target workspace is the imaging beam range, the performing obstacle processing on the target workspace comprises:
controlling the medical imaging system to pause medical image collection; and
in response to the obstacle being removed, allowing control of the medical imaging system to continue the medical image collection.

12. The method according to any one of claims 1 to 4, **characterized in that** the medical imaging system is an X-ray imaging system; and
the target subsystem comprises at least one of: an X-ray emission subsystem, an X-ray detection subsystem, and a patient support subsystem.

13. The method according to any one of claims 1 to 4, **characterized in that** the obstacle comprises at least one of the following types: other subsystems of the medical imaging system, a patient, a technician or medical personnel, and other objects in a space where the medical imaging system is located.

14. An obstacle processing apparatus for a medical imaging system, **characterized by** comprising:
a collection module, configured to collect, by means of a visible light camera, a camera image corresponding to a target workspace of a target subsystem of a medical imaging system, wherein the target workspace is a target movement path or an imaging beam range;
a detection module, configured to detect an obstacle in the target workspace based on the camera image to obtain an obstacle detection result of the target workspace; and
a processing module, configured to perform obstacle processing on the target workspace in response to the obstacle detection result indicating that an obstacle is detected.

15. An electronic device, **characterized by** comprising:
one or more processors; and
a memory for storing executable instructions,
wherein the one or more processors are configured to invoke the executable instructions stored by the memory to execute the method according to any one of claims 1 to 13.
